(19) **Europäisches Patentamt**

**European Patent Office**

**Office européen des brevets**

(11) **EP 0 811 396 B1**

(12) **FASCICULE DE BREVET EUROPEEN**

(45) Date de publication et mention
de la délivrance du brevet:
**21.04.2004 Bulletin 2004/17**

(51) Int Cl.⁷: **A61N 1/372**, H04L 25/03

(21) Numéro de dépôt: **97401241.1**

(22) Date de dépôt: **04.06.1997**

(54) **Dispositif de filtrage des signaux émis par un appareil médical, notamment un appareil médical actif implanté**

Vorrichtung zur Filterung von Signalen eines medizinischen Geräts, insbesondere eines aktiven implantierbaren medizinischen Geräts

Device for filtering the signals of a medical apparatus signal, in particular of an active implanted medical apparatus

(84) Etats contractants désignés:
**BE CH DE FR GB IT LI SE**

(30) Priorité: **04.06.1996 FR 9606823**

(43) Date de publication de la demande:
**10.12.1997 Bulletin 1997/50**

(73) Titulaire: **ELA MEDICAL (Société anonyme)**
**92541 Montrouge (FR)**

(72) Inventeurs:
• **Lee, Chik Yam**
**94110 - Arcueil (FR)**
• **Deschamp, Hervé**
**92150 - Suresnes (FR)**

(74) Mandataire: **Dupuis-Latour, Dominique et al**
**SEP Pagenberg & Associés**
**14 boulevard Malesherbes**
**75008 Paris (FR)**

(56) Documents cités:
**EP-A- 0 607 638          WO-A-91/16696**
**US-A- 4 494 545          US-A- 4 681 111**
**US-A- 4 864 590          US-A- 4 944 299**
**US-A- 5 181 228          US-A- 5 383 915**

• **SCHALDACH ET AL.: "Ein passives Telemetriesystem zur Überwachung von Herzschrittmacher-Elektroden" BIOMEDIZINISCHE TECHNIK, vol. 29, no. 9, septembre 1984, BERLIN, DE, pages 202-207, XP002025596**

## Description

La présente invention concerne le domaine des dispositifs médicaux, notamment les dispositifs médicaux implantés actifs, et plus particulièrement le recueil des signaux émis lors de séquences de communication entre le dispositif médical et une console de contrôle extérieure.

Les dispositifs médicaux implantés actifs comprennent notamment les stimulateurs cardiaques, les défibrillateurs, les appareils neurologiques, les pompes de diffusion de substance médicale, et les implants cochléaires.

L'invention n'est cependant pas limitée aux dispositifs médicaux implantés actifs et, bien que dans la suite on fera référence à ces derniers, l'invention s'applique aussi bien à des dispositifs médicaux non implantés (par exemple portés par le patient) ou non actifs (par exemple des dispositifs, implantés ou non, dépourvus de source d'énergie propre et utilisant pour l'émission de messages vers l'extérieur une fraction de l'énergie du signal d'interrogation qui leur est appliqué.

Ces appareils, une fois mis en place (notamment par implantation), sont programmés de l'extérieur au moyen d'une console distante appelée "programmateur". La vérification des paramètres de l'implant ou la transmission d'informations enregistrées par celui-ci s'effectuent par voie électromagnétique, appelée "télémétrie" dans la technique en question. Cette console est munie d'un organe récepteur qui est placé en regard du site de l'implant. Cet organe comprend une bobine qui capte le champ magnétique en provenance de l'appareil implanté.

Lorsque l'on opère ainsi une transmission d'informations de l'implant vers le programmateur, il y a lieu de tenir compte de la nature du matériau constituant le boîtier de l'implant. En effet, ce boîtier est généralement réalisé en un matériau métallique tel que le titane et ce matériau, du fait de ses propriétés conductrices de l'électricité, réémet une induction magnétique compensatrice qui fait l'effet d'un filtre passe-bas.

Ce filtrage passe-bas introduit une distorsion de type "interférence intersymbole" (ou encore "brouillage intersymbole" ou "interférence longitudinale") qui se traduit, dans une séquence de symboles (unités d'information) donnée, par un débordement d'un symbole sur le suivant. Pour des symboles adjacents, cette distorsion devient significative et peut introduire des ambiguïtés lors du décodage des symboles.

Ce problème est évoqué par le WO-A-91/16696, qui propose de modifier la fonction de transfert des circuits de réception du programmateur pour augmenter le débit des données tout en restant dans une plage de fonctionnement à l'écart de ce phénomène perturbateur.

L'un des buts de l'invention est de pallier la limitation précitée en proposant un circuit permettant de compenser la distorsion introduite par les boîtiers des implants, conduisant à une réduction significative notamment de l'interférence intersymbole et autorisant ainsi une augmentation significative du débit des signaux ou de la simplicité avec laquelle on va décoder les symboles.

À cet effet, l'invention propose d'équiper le programmateur d'un dispositif de filtrage recevant en entrée un signal délivré par un circuit de réception de signaux émis, sous forme d'une induction magnétique, par un appareil médical, cet appareil médical comprenant un boîtier métallique, dispositif caractérisé en ce qu'il comporte un étage de compensation possédant dans le domaine fréquentiel une fonction de transfert propre à inverser la perturbation introduite par la transmission du signal au travers du boîtier métallique, notamment une fonction de la forme :

$$\psi(f) = 1 + j\, \frac{f}{f_c} ,$$

où $f_c$ est un paramètre choisi en fonction de la géométrie et du matériau du boîtier métallique de l'appareil médical, et où $j = \sqrt{-1}$.

L'invention s'applique de manière particulièrement avantageuse au cas où l'appareil médical émet des messages formés de séquences de symboles élémentaires appartenant à un alphabet de n symboles (30-36), avec $n \geq 2$, l'étage de compensation étant alors un étage propre à réduire le taux d'interférence intersymbole des signaux reçus qui lui sont appliqués.

De préférence, il est prévu en outre un étage passe-bande dont les fréquences de coupure haute et basse sont choisies en fonction de la plage de fréquences des signaux émis par l'appareil implanté ; la fréquence de coupure haute et/ou la fréquence de coupure basse peuvent être des fréquences ajustables.

D'autres caractéristiques de l'invention apparaîtront à la lecture de la description détaillée ci-dessous faite en référence aux dessins annexés.

La figure 1 est un schéma par blocs de l'implant et des circuits de réception et de traitement des signaux du programmateur.

La figure 2 montre un exemple d'alphabet de quatre symboles pour réaliser la modulation du signal émis par l'implant.

La figure 3 illustre le phénomène d'interférence intersymbole dans le cas de l'utilisation de l'alphabet de la figure 2, en l'absence de filtrage selon l'invention.

La figure 4 est un schéma par blocs montrant les deux étages du dispositif de filtrage de l'invention.

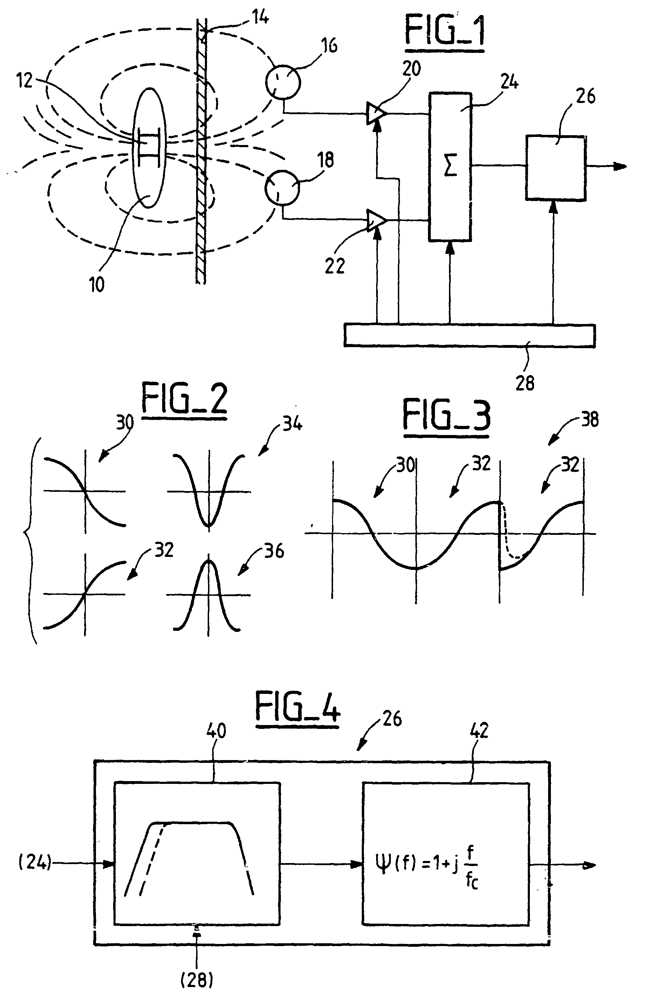

Sur la figure 1, la référence 10 désigne un implant, c'est-à-dire un "dispositif médical implanté actif" au sens large défini plus haut, en position opérante chez un patient. Cet implant contient en mémoire des informations qui sont par exemple véhiculées par l'induction magnétique provoquée par le passage de courants oscillants dans une bobine émettrice 12, permettant ainsi de transmettre par "télémétrie" ces informations au-delà de la barrière cutanée 14.

Les signaux ainsi émis, qui sont de nature essentiellement magnétique comme expliqué plus haut, sont recueillis par l'organe récepteur d'un programmateur, qui est par exemple un dispositif d'un type comparable à celui décrit dans le EP-A-0 661 077 auquel on pourra se reporter pour de plus amples détails sur la manière dont les signaux sont recueillis et traités.

On peut en particulier (mais de manière non limitative à l'égard de l'invention), comme décrit dans ce document, extraire du signal total véhiculé sous forme de champ magnétique la seule composante de signal utile en éliminant la quasi-totalité de la composante de signal parasite. À cet effet, on prévoit une pluralité de bobines distinctes telle qu'une bobine collectrice 16 et une bobine de compensation 18, recueillant chacune des flux magnétiques induits propres et produisant des tensions correspondantes appliquées à des amplificateurs respectifs 20, 22 à gain variable, dont les sorties sont combinées entre elles dans un étage sommateur 24.

En sortie de l'étage sommateur 24 on peut obtenir par un ajustement approprié des gains des amplificateurs 20 et 22 un signal essentiellement constitué de la seule composante du signal utile.

L'invention propose un circuit 26 permettant de traiter la composante utile de manière à réduire une certaine forme de distorsion que l'on va exposer plus bas.

Ce circuit 26, comme d'ailleurs les autres circuits de traitement et signaux de programmateur, peut être réalisé de différentes manières, par exemple de manière entièrement analogique, ou bien avec une partie de circuit numérique plus ou moins importante, pilotée ou non par des moyens logiciels. Les différents blocs fonctionnels sont pilotés par un circuit de commande 28 qui peut avantageusement comporter un microcalculateur permettant d'ajuster les paramètres des différents éléments du circuit pour obtenir les résultats cherchés. En tout état de cause, la réalisation matérielle de ces circuits n'est en aucune façon limitative à l'égard des enseignements de l'invention, et elle est d'ailleurs en elle-même du domaine de l'homme de l'art ; pour cette raison, ces aspects matériels ne seront pas décris plus en détails

On va maintenant expliquer les phénomènes de distorsion auxquels le circuit de l'invention peut remédier.

Le boîtier de l'implant 10 est typiquement réalisé en matériau métallique, le plus souvent le titane ou un alliage à base de titane (ce matériau particulier n'est bien entendu pas limitatif à l'égard de la présente invention).

Comme l'induction magnétique est produite par une source (bobine émettrice 12) qui est enfermée dans un boîtier conducteur, le signal utile au sens indiqué plus haut (c'est-à-dire le signal débarrassé des parasites provenant de sources magnétiques d'origine externe) va se trouver perturbé, ce qui se traduira par une atténuation du haut du spectre dans le domaine fréquentiel, c'est-à-dire un filtrage passe-bas.

On va expliquer ce phénomène particulier d'interférence intersymbole en particulier en référence aux figures 2 et 3.

On prendra l'exemple d'une modulation quaternaire réalisée par combinaison d'une modulation binaire de phase et d'une modulation binaire de fréquence (mais le problème se pose en des termes comparables par exemple pour une modulation de phase à quatre états, ou pour d'autres types de modulation encore).

On définit ainsi un "alphabet" de quatre "symboles" 30, 32, 34 et 36 illustrés figure 2.

Les symboles 30, 32 d'une part et 34, 36 d'autre part sont émis à deux fréquences différentes, par exemple 8 et 16 kHz, tandis que les symboles 30, 34 d'une part et 32, 36 d'autre part sont émis avec un déphasage respectif de 180° par rapport à 30 et 34.

Si l'on considère par exemple, comme illustré figure 3, la séquence 38 formée d'une succession de trois symboles 30, 32 et 32, si la transition entre les symboles 30 et 32 se fait sans solution de continuité, en revanche la transition entre les symboles 32 successifs se fait avec une discontinuité brusque et importante du niveau du signal, correspondant à un passage abrupt d'un niveau positif à un niveau négatif. Cette transition abrupte se traduit dans le domaine fréquentiel par la production de composantes de rang élevé. Or, comme on l'a expliqué plus haut, la présence du boîtier en titane joue un rôle de filtre passe-bas naturel qui empêche la transmission de ces harmoniques de rang élevé, introduisant de ce fait une distorsion du signal qui, dans la réalité, prendra la forme illustrée en tiretés sur la figure 3.

Si l'on compare les figures 2 et 3, on s'aperçoit que la forme d'onde réellement émise depuis l'implant, et donc captée par le programmateur, est une forme d'onde qui est intermédiaire entre celle du symbole 32 et celle du symbole 34.

Ce phénomène est connu sous le nom d'"interférence intersymbole" (ou encore "interférence longitudinale" ou "brouillage intersymbole"), phénomène qui peut être défini simplement comme le "débordement" d'un symbole sur le suivant.

On notera que cette distorsion est intrinsèque au signal émis par la bobine, et apparaît donc sur le signal utile "pur", c'est-à-dire débarrassé de tous les parasites d'origine externe. En d'autres termes, l'amélioration du rapport signal/bruit dans la chaîne de réception et de traitement du signal est sans incidence sur ce type de distorsion qui, si elle atteint un niveau excessif, peut introduire des ambiguïtés pouvant aller jusqu'à des erreurs dans la transmission des symboles (le troisième symbole de la séquence 38 de la figure 3 pouvant par exemple être interprété comme un symbole 34 et non comme un symbole 32).

De plus, l'effet de filtrage passe-bas naturel du titane, intrinsèque au matériau, est d'autant plus accentué que la fréquence du signal est élevée.

L'invention vise précisément à réduire ce phénomène avec pour conséquences principales (entre autres), d'une part, la réduction considérable de l'interférence intersymbole et donc la diminution, voir la suppression, du risque d'ambiguïté lors du décodage des symboles.

L'invention part du fait que, lorsqu'une induction magnétique est produite par une source enfermée dans un boîtier conducteur, dans le domaine des fréquences cette induction se voit multipliée à l'extérieur du boîtier par une fonction de transfert de la forme :

$$\chi(f) = \frac{1}{1 + j\dfrac{f}{f_c}} \,,$$

où $f_c$ est un paramètre dépendant de la géométrie et du matériau du boîtier métallique, et où $j = \sqrt{-1}$.

Pour restituer, à partir de la composante utile du signal capté, un signal correspondant au signal non perturbé par le boîtier, on peut prévoir un élément dont la fonction de transfert $\psi(f)$, inverse de $\chi(f)$, compensera cette dernière :

$$\psi(f) = 1 + j\,\frac{f}{f_c} \,.$$

Le dispositif de filtrage 26 de l'invention peut être constitué, comme illustré sur la figure 4, d'un premier étage 40 de filtrage, formé d'un filtre passe-bande, suivi d'un étage 42 de compensation, de fonction de transfert $\psi(f)$.

L'étage de filtrage 40 (qui n'est pas en soi en tant que tel indispensable à la mise en oeuvre de l'invention, mais qui en améliore avantageusement l'efficacité) est un filtre passe-bande en lui-même classique, de préférence de type programmable à gabarit réglable par une commande appropriée en provenance du circuit de pilotage 28. Un tel circuit programmable permet notamment de prendre en compte automatiquement des signaux utiles émis sur des fréquences porteuses différentes, par exemple pour permettre au programmateur de lire des données en provenance aussi bien d'implants classiques fonctionnant à des fréquences de 8 à 16 kHz que d'implants de type nouveau fonctionnant à des fréquences plus élevées, ou encore des données en provenance d'implants de marques différentes.

Le filtrage passe-bande est en tout état de cause souhaitable pour améliorer le rapport signal/bruit du signal traité en éliminant des composantes fréquentielles indésirables situées hors de la bande passante normale.

Les fréquences de coupure basse et haute peuvent être par exemple de 90 kHz et 160 kHz respectivement, pour traiter un signal utile dont la fréquence porteuse est de 128 kHz (par exemple avec des implants de type nouveau à fréquence porteuse élevée) ou bien 4 kHz et 160 kHz respectivement, pour traiter un signal utile de fréquence porteuse allant de 8 kHz à 16 kHz, comme c'est le cas avec les implants typiques actuels.

Le signal ainsi filtré par l'étage 40 est alors transmis à l'étage de compensation 42 appliquant la fonction de transfert $\psi(f)$ permettant de restaurer le profil du train de symboles, en restituant la forme (ou à tout le moins une forme très proche) que celui-ci aurait s'il avait été émis par une bobine non enfermée dans un boîtier métallique.

## Revendications

1. Un dispositif coopérant avec un appareil médical (10), cet appareil médical comprenant un boîtier métallique et émettant un signal sous forme d'une induction magnétique, ce signal étant déformé par sa transmission au travers du boîtier métallique, dispositif **caractérisé en ce qu'**il comprend, pour rétablir le signal déformé transmis, un étage de compensation (42) possédant dans le domaine fréquentiel une fonction de transfert propre à inverser la perturbation introduite par la transmission du signal au travers du boîtier métallique.

2. Le dispositif de la revendication 1, dans lequel la fonction de transfert est de la forme :

$$\psi(f) = 1 + j\,\frac{f}{f_c} \,,$$

où $f_c$ est un paramètre choisi en fonction de la géométrie et du matériau du boîtier, et où $j = \sqrt{-1}$.

3. Le dispositif de la revendication 2, dans lequel l'appareil médical émet des messages formés de séquences de symboles élémentaires appartenant à un alphabet de n symboles (30-36), avec $n \geq 2$, l'étage de compensation étant alors un étage propre à réduire le taux d'interférence intersymbole des signaux reçus qui lui sont appliqués.

4. Le dispositif de la revendication 2, dans lequel il est prévu en outre un étage passe-bande (40) dont les fréquences de coupure haute et basse sont choisies en fonction de la plage de fréquences des signaux émis par l'appareil implanté.

5. Le dispositif de la revendication 3, dans lequel la fréquence de coupure haute et/ou la fréquence de coupure basse sont des fréquences ajustables.

## Claims

1. An apparatus for co-operating with a medical device (10), said medical device comprising a metallic case and emitting a signal in the form of a magnetic induction, said signal being distorted by the transmission thereof through the metallic case, said apparatus being **characterised in that** it comprises, in order to restore the distorted signal transmitted, a compensation stage (42) having in the frequency domain a transfer function which is adapted to invert the disturbance introduced by the transmission of the signal through the metallic case.

2. The apparatus of claim 1, wherein the transfer function is of the form:

$$\psi(f) = 1 + j\,\frac{f}{f_c}\,,$$

where $f_c$ is a parameter chosen according to the geometry and the material of the metallic case, and where $j=\sqrt{-1}$.

3. The apparatus of claim 2, wherein the medical device transmits messages comprised of sequences of elementary symbols belonging to an alphabet of n symbols (30-36), where $n \geq 2$, the compensation stage being in this case a stage adapted to reduce the rate of inter-symbol interference of collected signals that are applied to it.

4. The apparatus of claim 2, wherein there is further provided a band-pass filter stage (40) which high and low cut-off frequencies are selected depending on the frequency interval of the signals transmitted by the implanted device.

5. The apparatus of claim 3, wherein the high cutoff frequency and/or the low cutoff frequency are adjustable frequencies.

## Patentansprüche

1. Vorrichtung, die mit einem medizinischen Apparat (10) zusammenwirkt, wobei der medizinische Apparat ein metallisches Gehäuse umfasst und ein Signal aussendet, in Form einer magnetischen Induktion, wobei das Signal wegen seiner Übertragung durch das metallische Gehäuse deformiert wird, **dadurch gekennzeichnet, dass** die Vorrichtung, um das deformierte übertragene Signal wiederherzustellen, eine Kompensationsstufe (42) umfasst, welche in dem Frequenzbereich eine Übertragungsfunktion besitzt, die geeignet ist, die Störung umzukehren, welche durch die Übertragung des Signals durch das metallische Gehäuse eingeführt wurde.

2. Vorrichtung gemäß Anspruch 1, in welchem die Übertragungsfunktion von der Form ist:

$$\psi(f) = 1 + j\,\frac{f}{f_c}$$

wobei $f_c$ ein Parameter ist, der in Abhängigkeit von der Geometrie und dem Material des Gehäuses gewählt wurde, und wo $j = \sqrt{-1}$.

3. Vorrichtung gemäß Anspruch 2, in welchem der medizinische Apparat Nachrichten aussendet, die aus Sequenzen von elementaren Symbolen geformt sind, die zu einem Alphabet von n Symbolen (30 bis 36) gehören, mit $n \geq 2$, wobei die Kompensationsstufe somit eine Stufe ist, welche geeignet ist, den Grad der Intersymbol-Interferenzen von empfangenen Symbolen zu reduzieren, die an diese angelegt werden.

4. Vorrichtung gemäß Anspruch 2, in welchem ferner eine Bandpassstufe (40) vorgesehen ist, deren obere und untere Grenzfrequenzen in Abhängigkeit von den Frequenzbereichen der durch den eingebauten Apparat ausgesendeten Frequenzen gewählt sind.

5. Vorrichtung gemäß Anspruch 3, in welchem die obere Grenzfrequenz und/oder die untere Grenzfrequenz anpassbare Frequenzen sind.

FIG_1

FIG_2

FIG_3

FIG_4

$$\Psi(f) = 1 + j\,\dfrac{f}{f_C}$$